Europäisches Patentamt

European Patent Office

Office européen des brevets

(19)

(11) Numéro de publication: **0 490 003 B1**

## (12) FASCICULE DE BREVET EUROPEEN

(45) Date de publication de fascicule du brevet: **15.02.95**

(51) Int. Cl.6: **C07C 19/08**, C07C 17/38, C07C 21/08

(21) Numéro de dépôt: **90403440.2**

(22) Date de dépôt: **04.12.90**

---

(54) **Procédé pour éliminer le dichloroacétylène du 1,1-dichloro-1-fluoroéthane et/ou du chlorure de vinylidène.**

---

(30) Priorité: **14.12.89 US 451266**

(43) Date de publication de la demande:
**17.06.92 Bulletin 92/25**

(45) Mention de la délivrance du brevet:
**15.02.95 Bulletin 95/07**

(84) Etats contractants désignés:
**BE DE ES FR GB GR IT NL**

(56) Documents cités:
EP-A- 0 389 334      GB-A- 846 677
GB-A- 1 447 452      GB-A- 1 453 509
US-A- 2 894 044      US-A- 4 906 796

(73) Titulaire: **ELF ATOCHEM S.A.**
**4 & 8, Cours Michelet**
**La Défense 10**
**F-92800 Puteaux (FR)**

(72) Inventeur: **Yates, Stephen Frederic**
**2140 Goebbert Road No. 4-210**
**Arlington Heights,**
**Illinois 60005 (US)**

(74) Mandataire: **Leboulenger, Jean et al**
**ATOCHEM**
**Département Propriété Industrielle**
**F-92091 Paris la Défense 10 Cédex 42 (FR)**

**Description**

Cette invention concerne la purification du 1,1-dichloro-1-fluoroéthane, désigné aussi par CFC-141b, qui présente un intérêt particulier comme remplaçant des chlorofluorocarbures ayant des propriétés physiques semblables, en particulier le CFC-11, le CFC-12 et le CFC-113. Le CFC-141b peut être préparé par réaction de chlorure de vinylidène ou de trichloroéthane avec HF. Ces procédés sont décrits, par exemple, dans les brevets U.S. 2 894 044 et 3 833 676.

Il est caractéristique de ces réactions qu'il se forme de nombreux sous-produits contenant des nombres différents d'atomes d'hydrogène, de chlore et de fluor sur les molécules de méthane, d'éthane et d'éthylène. Lorsque cela est possible, on peut séparer par distillation ces sous-produits et la matière première n'ayant pas réagi. D'autres composés sont relativement inoffensifs, car leur présence n'altère pas beaucoup les propriétés physiques pour lesquelles le CFC-141b est utile. Un contaminant qui doit être éliminé à cause de sa toxicité est le dichloroacétylène, bien que seules des quantités relativement faibles soient en général présentes dans le CFC-141b formé et on pense que celles-ci étaient déjà présentes dans le chlorure de vinylidène utilisé.

Il est souhaitable d'avoir une autre amélioration des procédés de purification du CFC-141b, notamment pour ce qui est de l'élimination du dichloroacétylène, et le présent inventeur a découvert un moyen de purification par adsorption qui va être décrit en détail ci-dessous.

RESUME DE L'INVENTION

Le dichloroacétylène peut être éliminé pratiquement complètement d'un courant de CFC-141b contenant initialement environ 4 à 20 ppm en poids de dichloroacétylène. On fait passer le courant de CFC-141b sur un tamis moléculaire carboné ayant une grosseur moyenne de pore d'environ 4,2 à 4,5 angströms à une température d'environ -20°C à 60°C et sous une pression d'environ 100 à 500 kPa. L'élimination du dichloroacétylène est essentiellement complète, autrement dit inférieure à la limite de détection de 2 ppm en poids. En variante, on peut éliminer le dichloroacétylène du chlorure de vinylidène avant de l'introduire dans le procédé de fluoration.

Le procédé peut être réalisé avec du CFC-141b (ou avec du chlorure de vinylidène) en phase liquide ou vapeur. Lorsqu'on utilise un lit fixe de particules de tamis moléculaire, on fait passer de la vapeur de CFC-141b (ou de chlorure de vinylidène) sur les particules avec une vitesse spatiale horaire de gaz d'environ 130 à 1500 h$^{-1}$. La vitesse spatiale de liquide correspondante pour une opération en phase liquide serait d'environ 1 à 15 h$^{-1}$.

DESCRIPTION DES DESSINS

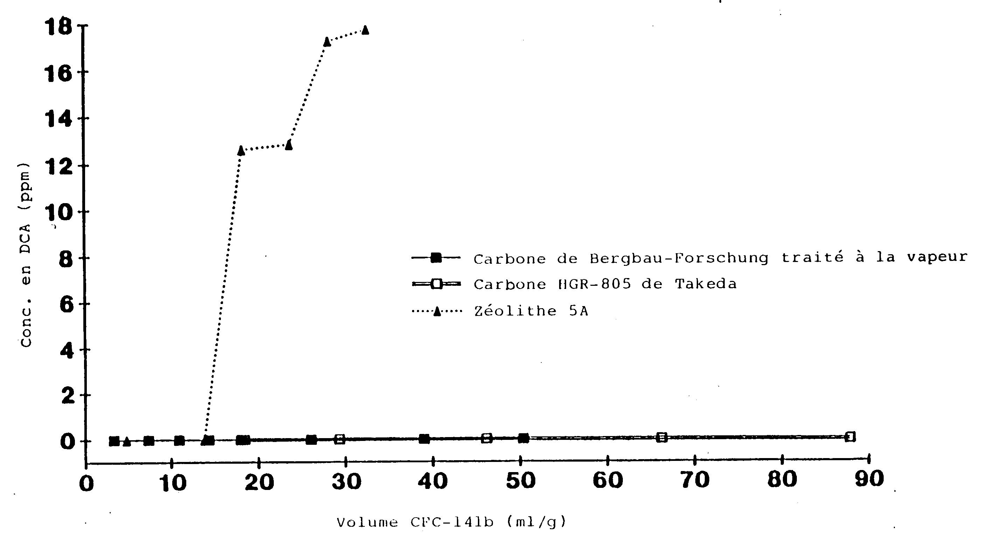

La seule figure est un graphique indiquant l'adsorption du dichloroacétylène sur divers tamis moléculaires carbonés.

Description des modes de réalisation préférés

Adsorption du dichloroacétylène

Le dichloroacétylène est présent dans le CFC-141b en quantités comprises entre 4 et 20 ppm en poids et peut provenir du chlorure de vinylidène introduit dans la réaction de fluoration.

On préfère éliminer sélectivement un contaminant comme le dichloroacétylène du CFC-141b. On utilise couramment une distillation, mais elle n'est pas utilisable pour éliminer de faibles quantités de dichloroacétylène. On peut envisager l'adsorption pour ces applications. Toutefois, il est possible qu'un adsorbant élimine plus que le composé cible, à savoir le dichloroacétylène, ce qui augmente son coût d'élimination et, lorsque l'adsorbant sera régénéré, le composé cible sera contaminé par d'autres composés. Cependant, le présent inventeur a découvert qu'en choisissant l'adsorbant de type tamis moléculaire adéquat, on peut arriver à une séparation quasi idéale du dichloroacétylène d'avec le CFC-141b.

Comme le montrent les exemples ci-dessous, le dichloroacétylène n'est pas éliminé dans une mesure significative par de nombreux tamis moléculaires. Toutefois, on a constaté que les tamis moléculaires carbonés ayant une grosseur moyenne de pore d'environ 4,2 à 4,5 angströms étaient utiles.

2

Tamis moléculaires carbonés

Les tamis moléculaires carbonés sont disponibles dans le commerce. Ils sont habituellement dérivés de sources naturelles comme le charbon. Un exemple est les tamis moléculaires carbonés décrits dans un article de Juntgen et coll. de Bergbau-Forschung GmbH dans FUEL, 1981, vol. 60, septembre, p. 817-822.

Un autre tamis moléculaire carboné utilisé pour la purification des hydrocarbures fluorés est produit par un procédé particulier décrit dans le brevet U.S. 4 820 681 et cité ici à titre de référence. Ce procédé de fabrication peut être en gros caractérisé comme comprenant trois étapes: (1) polymérisation d'un monomère non oxygéné en présence d'un agent de réticulation non oxygéné; (2) formation de particules du polymère résultant en la forme souhaitée; et puis (3) carbonisation du matériau formé dans un environnement essentiellement non oxygéné.

Le monomère peut être choisi parmi un certain nombre de monomères différents. Ils doivent être facilement polymérisables, essentiellement non oxygénés dans leur structure moléculaire et de préférence constitués fondamentalement d'hydrogène, d'un halogène et de carbone. Parmi les substances que l'on peut employer comme monomère figurent l'acrylonitrile (AN), le fluorure de vinylidène (PVDF), le chlorotrifluoroéthylène (HALAR), le chlorure de vinylidène (PVCD), des mélanges de deux monomères ou plus, comme les mélanges de chlorure de vinylidène et de chlorure de vinyle, de chlorure de vinylidène et d'acrylonitrile, et un mélange de styrène et de divinylbenzène. D'autres monomères convenables sont le fluorure de vinyle, le bromure de vinyle, l'éthylène chloré, le chlorofluoroéthylène, le vinylchlorobenzène, le bromure de vinylidène et le fluorure de vinylidènechlorotrifluoroéthylène. Le monomère préféré est le chlorure de vinylidène. Les réactions de polymérisation peuvent être réalisées selon un certain nombre de procédés différents connus dans la technique. Toutefois, on a obtenu les résultats les plus intéressants en employant une polymérisation en masse ou une polymérisation en solution.

Les polymères produits dans l'étape de polymérisation initiale doivent être réticulés avec un agent de réticulation essentiellement non oxygéné. L'agent de réticulation sera typiquement présent pendant la polymérisation en une concentration égale ou inférieure à 10% en moles du monomère. Un agent de réticulation préféré est le divinylbenzène. D'autres agents de réticulation possibles englobent le trivinylbenzène, le divinylacétylène et le sulfure de divinyle.

Comme on souhaite produire des tamis moléculaires carbonés à partir de polymères ne comportant pas de fonctions oxygénées, l'initiateur de polymérisation est aussi de préférence un composé non oxygéné. Par conséquent, on utilise de préférence un initiateur carboné ou azoïque plutôt qu'oxygéné.

La substance polymère est carbonisée par chauffage jusqu'à une température élevée dans un environnement essentiellement non oxygéné. Avant la carbonisation à haute température, la substance précurseur est soumise à une étape de chauffage ménagé au cours de laquelle sa température est élevée au-dessus de 150°C, par exemple à 240°C, et maintenue à cette valeur jusqu'à ce que l'on n'observe plus de perte de poids. La substance est ensuite de préférence soumise à une élévation programmée de température jusqu'à une température supérieure à 700°C, de préférence supérieure à 800°C, en particulier supérieure à 900°C. Les précurseurs de tamis dérivés de substances polymères sont essentiellement exempts des substances minérales comme les métaux et les oxydes minéraux qui peuvent être présents lorsque la substance précurseur est faite d'une substance naturelle comme le charbon, les écorces de noix de coco, la tourbe ou le bois. Les tamis préférés, sur une base non hydrogénée et non oxygénée, doivent contenir au moins 99,5% en poids de carbone et de préférence au moins 99,8% en poids de carbone.

Tandis que le procédé que l'on vient de décrire produit un tamis moléculaire carboné spécial et utile, on pense que sa grosseur moyenne de pore est légèrement supérieure à 3,8 angströms et, par conséquent, on doit encore le traiter pour accroître sa grosseur de pore afin qu'il satisfasse aux limites de grosseur de pore requises. On peut utiliser diverses techniques pour augmenter la grosseur des pores, comme un traitement avec de la vapeur d'eau à des températures comprises entre environ 700°C et 1000°C, un traitement avec de l'air à des températures comprises entre environ 400°C et 600°C, ou un traitement avec $CO_2$ à des températures comprises entre environ 700°C et 1000°C.

On notera qu'il est difficile de déterminer la grosseur de pore de tamis moléculaires carbonés et que, par conséquent, on ne dispose pas toujours de valeurs précises. Plusieurs approches ont été utilisées. Dans le premier procédé, on met en contact une série de molécules de taille croissante avec le tamis moléculaire carboné et on mesure la quantité adsorbée dans une balance de McBain. Lorsque la quantité d'une molécule adsorbée est sensiblement supérieure à celle trouvée avec d'autres molécules, on considère que la grosseur de pore a été déterminée. Dans le deuxième procédé, on étudie le comportement d'un mélange de gaz en utilisant un tamis moléculaire carboné comme adsorbant chromatographique. On estime la grosseur des pores en observant lequel de ces gaz est retenu sur l'adsorbant. Un autre procédé encore nécessite la mesure de la chaleur isostérique d'adsorption d'un ou de plusieurs gaz. La grosseur des pores est donnée par l'intersection d'une ligne tracée à cette énergie avec la courbe de potentiel de Lennard-Jones pour ce gaz. Un exemple de cette dernière technique est donné par K. Chihara et coll., dans le Journal of Colloids and Interface Science, 64, 584 (1978), dans lequel on trouve que la grosseur des pores du tamis moléculaire MSC-5A est de 4,4 Å.

Procédé

Lorsqu'on produit le CFC-141b par hydrofluoration catalytique du chlorure de vinylidène, la conversion en CFC-141b n'est que partielle et de nombreux sous-produits sont produits. Par conséquent, l'effluent du réacteur est séparé par distillation pour concentrer le produit CFC-141b et pour produire un courant de recyclage de la charge n'ayant pas réagi. Le courant de CFC-141b impur qui en résulte contient du HF et du chlorure de vinylidène n'ayant pas réagi, ainsi que des quantités mineures d'impuretés comme sous-produits, y compris du dichloroacétylène qui peut être présent dans la charge de chlorure de vinylidène. Le HF et le HCl peuvent être éliminés sélectivement par une technique décrite par d'autres et ne faisant pas partie de la présente invention. Une fois cela fait, le CFC-141b contient encore des impuretés qui doivent être éliminées, en particulier le dichloroacétylène qui est toxique et qui doit être éliminé. Le présent procédé a pour but d'être sélectif pour éliminer le dichloroacétylène jusqu'à une proportion inférieure ou égale à 2 ppm en poids dans un courant de CFC-141b concentré.

Le traitement du CFC-141b peut être réalisé en phase liquide ou en phase gazeuse, bien que l'on préfère la phase liquide pour éviter le coût de la vaporisation et de la condensation ultérieure. Diverses techniques connues de l'homme de métier peuvent être utilisées pour mettre en contact le courant de CFC-141b avec le tamis moléculaire carboné formant l'adsorbant, comme des lits fluides ou mobiles, mais, typiquement, on utilise un lit fixe de particules adsorbantes. Le choix de la granulométrie, de la forme du lit et de la vitesse spatiale du courant de CFC-141b est déterminé suivant les principes connus, selon les exigences pour obtenir l'élimination quasi totale souhaitée du dichloroacétylène. Généralement, la vitesse spatiale horaire de gaz du courant de CFC-141b est d'environ 130 à 1500 $h^{-1}$ lorsqu'on opère en phase gaz. La vitesse spatiale de liquide correspondante serait d'environ 1 à 15 $h^{-1}$. L'adsorption est réalisée à une température convenable, généralement comprise entre environ -20°C et 60°C et sous une pression, qui dépend si l'on souhaite une mise en contact liquide ou vapeur, d'environ 100 à 500 kPa. Comme le montrent les exemples ci-dessous, on peut prévoir que le dichloroacétylène sera presque entièrement éliminé, laissant 2 ppm en poids ou moins dans le courant de CFC-141b prêt pour une purification ultérieure éventuelle.

Dans un autre mode de réalisation, le dichloroacétylène est éliminé avant la fluoration du chlorure de vinylidène par passage du courant de charge sur les tamis moléculaires carbonés de la manière que l'on a indiquée ci-dessus pour le CFC-141b.

Le lit adsorbant doit fournir une capacité optimale pour le dichloroacétylène, en faisant le bilan des coûts de l'installation et de l'adsorbant par rapport aux coûts de régénération. Lorsqu'on a atteint la capacité utile, on régénère l'adsorbant en chauffant le lit avec un courant gazeux pour éliminer le dichloroacétylène. L'analyse des gaz effluents suggère que le dichloroacétylène est décomposé pendant le chauffage, car on n'en décèle pas. Le CFC-141b restant dans le récipient et sur l'adsorbant est d'abord séparé et récupéré, puis le procédé de régénération est mis en oeuvre. Après avoir totalement chauffé le lit, on le refroidit et on le réintroduit dans le circuit. Les conditions nécessaires pour régénérer de façon optimale l'adsorbant seront déterminées en fonction de l'adsorbant utilisé et des utilitésdisponibles. Typiquement, on prévoit qu'un chauffage du lit d'adsorbant à environ 200°C-500°C avec un courant d'azote produira une régénération satisfaisante.

Exemple 1

On étudie la capacité d'adsorption du dichloroacétylène d'un certain nombre d'adsorbants potentiels. On place dans une fiole de 20 ml 1,0 g de l'adsorbant à étudier et un échantillon de 15 ml de CFC-141b impur contenant 576 ppm en poids de chlorure de vinylidène, 16 ppm en poids de dichloroacétylène, 840 ppm en poids de CFC-142b (1-chloro-1,1-difluoroéthane) et 20 ppm en poids de CFC-1131a (1-chloro-1-fluoroéthylène). Après une heure d'agitation, on prélève un échantillon du liquide et on l'analyse par chromatographie en phase gazeuse en utilisant deux colonnes en acier inoxydable en série de diamètre 3,175 mm (6,1 m de n-octane-Porasil C et 2 m de OV-101 à 10% sur Chromosorb W, ces deux substances étant de 80/100 mesh, disponibles auprès de Alltech Associates) et 18 ml/min d'azote comme gaz vecteur. Les résultats figurent sur le tableau ci-dessous.

## Tableau 1

| Adsorbant | Dichloroacétylene ppm en poids |
|---|---|
| Charge (sans adsorbant) | 16 |
| Chabazite (AW-500)[a] | < 2 |
| 5A[b] | < 2 |
| 3A[c] | 19 |
| Calcium X[d] | 19 |
| Mordénite (AW-300)[e] | 2,6 |
| Tamis mol. carboné[f] | 2,3 |
| Tamis mol. carboné[g] | < 2 |
| Tamis mol. carboné[h] | < 2 |
| Tamis mol. carboné[i] | < 2 |

(a) fourni par UOP

(b) fourni par UOP

(c) fourni par UOP

(d) fourni par UOP

(e) fourni par UOP

(f) préparé par le procédé du brevet U.S. 4 820 681 au moyen de poly(chlorure de vinylidène) carbonisé à 800°C

(g) fourni par Takeda Chemical Co. (MSC-5A)

(h) fourni par Bergbau-Forschung GmbH

(i) fourni par Bergbau-Forschung GmbH, puis traité à la vapeur d'eau à 850°C pendant 30 minutes.

Le CFC-142b et le CFC-1131a ne sont pratiquement pas éliminés.

On constate que la plupart des adsorbants n'adsorbent pas le dichloroacétylène. Le 5A et la chabazite sont efficaces, mais le 5A a une faible capacité (voir exemple 3), tandis que la chabazite semble réagir avec le CFC-141b. Les tamis moléculaires carbonés sont d'efficacités égales, mais ne présentent pas ces inconvénients. On notera que le tamis moléculaire (f), qui a une grosseur de pore légèrement plus grande que 3,8 Å, est moins efficace que les tamis (g), (h) et (i), qui ont des grosseurs moyennes de pore entre 4,2 et 4,5 Å.

Exemple 2

On pompe du chlorure de vinylidène contenant 8,2 ppm de dichloroacétylène à 0,88 ml/min dans une colonne de diamètre 9,5 mm et de 177,8 mm de longueur contenant un tamis moléculaire carboné broyé à 20-50 mesh. La colonne est chargée avec 6,125 g d'un tamis moléculaire carboné fourni par Bergbau-Forschung ayant été traité à la vapeur d'eau à 850°C. On prélève périodiquement des échantillons du produit que l'on analyse par la méthode analytique de l'exemple 1. Les résultats de cette analyse sont indiqués sur le tableau ci-dessous.

## Tableau 2

| Echantillon | Volume élué (ml) | Conc. en dichloroacétylène |
|---|---|---|
| 1 | 44 | aucun décelé |
| 2 | 77 | aucun décelé |
| 3 | 145 | 4,9 ppm |

Exemple 3

On pompe du CFC-141b contenant 8,5 ppm de dichloroacétylène à 0,88 ml/min dans une colonne de mêmes dimensions que dans l'exemple 2 et contenant 2-10 g de zéolithe 5A, de tamis moléculaire carboné fourni par Takeda Chemical Co. (HGR-805) ou de tamis moléculaire carboné fourni par Bergbau-Forschung ayant été ensuite traité à la vapeur d'eau à 850°C pendant 30 min. On prélève périodiquement des échantillons du produit que l'on analyse en utilisant la méthode analytique de l'exemple 1. Les résultats de cette analyse sont indiqués sur la figure. On constate que le dichloroacétylène traverse rapidement lorsque le 5A est l'adsorbant, mais que l'on ne décèle absolument aucun dichloroacétylène lorsqu'on utilise l'un ou l'autre des tamis moléculaires carbonés.

## Revendications

1. Procédé de purification de 1,1-dichloro-1-fluoroéthane (CFC-141b) contenant environ 4-20 ppm en poids de dichloroacétylène, comprenant le passage dudit 1,1-dichloro-1-fluoroéthane sur un tamis moléculaire carboné ayant une grosseur moyenne de pore comprise entre environ 4,2 et 4,5 angströms, à une température d'environ -20°C à 60°C et sous une pression d'environ 100 à 500 kPa et la récupération de 1,1-dichloro-1-fluoroéthane contenant moins de 2 ppm en poids de dichloroacétylène.

2. Procédé selon la revendication 1, dans lequel ledit tamis moléculaire carboné est un lit fixe de particules, le 1,1-dichloro-1-fluoroéthane est un gaz et la vitesse spatiale horaire de gaz dudit 1,1-dichloro-1-fluoroéthane est d'environ 130 à 1500 $h^{-1}$.

3. Procédé selon la revendication 1, dans lequel ledit tamis moléculaire carboné est un lit fixe de particules, le 1,1-dichloro-1-fluoroéthane est un liquide et la vitesse spatiale horaire de liquide dudit 1,1-dichloro-1-fluoroéthane est d'environ 1 à 15 $h^{-1}$.

4. Procédé de purification de chlorure de vinylidène contenant environ 4-20 ppm en poids de dichloroacétylène, comprenant le passage dudit chlorure de vinylidène sur un tamis moléculaire carboné ayant une grosseur moyenne de pore comprise entre environ 4,2 et 4,5 angströms, à une température de -20°C à 60°C et sous une pression d'environ 100 à 500 kPa et la récupération de chlorure de vinylidène contenant moins de 2 ppm en poids de dichloroacétylène.

5. Procédé selon la revendication 4, dans lequel ledit tamis moléculaire carboné est un lit fixe de particules, le chlorure de vinylidène est un gaz et la vitesse spatiale horaire de gaz dudit chlorure de vinylidène est d'environ 130 à 1500 $h^{-1}$.

6. Procédé selon la revendication 4, dans lequel ledit tamis moléculaire carboné est un lit fixe de particules, le chlorure de vinylidène est un liquide et la vitesse spatiale horaire de liquide dudit chlorure de vinylidène est d'environ 1 à 15 $h^{-1}$.

## Claims

1. Process for purification of 1,1-dichloro-1-fluoroethane (CFC-141b) containing approximately 4 to 20 ppm by weight of dichloroacetylene, comprising passing the said 1,1-dichloro-1-fluoroethane over a carbonaceous molecular sieve which has a mean pore size of between approximately 4.2 and 4.5 angstroms, at a temperature of -20°C to 60°C and at a pressure of approximately 100 to 500 kPa and recovering 1,1-dichloro-1-fluoroethane containing less than 2 ppm by weight of dichloroacetylene.

2. Process according to claim 1, in which the said carbonaceous molecular sieve is a stationary bed of particles, 1,1-dichloro-1-fluoroethane is a gas and the hourly gas space velocity of the said 1,1-dichloro-1-fluoroethane is from approximately 130 to 1500 $h^{-1}$.

3. Process according to claim 1, in which the said carbonaceous molecular sieve is a stationary bed of particles, 1,1-dichloro-1-fluoroethane is a liquid and the hourly liquid space velocity of the said 1,1-dichloro-1-fluoroethane is from approximatelyl 1 to 15 $h^{-1}$.

4. Process for purification of vinylidene chloride containing approximately 4 to 20 ppm by weight of dichloroacetylene, comprising passing the said vinylidene chloride over a carbonaceous molecular sieve which has a mean pore size of between approximately 4.2 and 4.5 angstroms, at a temperature of -20°C to 60°C and at a pressure of approximatively 100 to 500 kPa and recovering vinylidene chloride containing less than 2 ppm by weight of dichloroacetylene.

5. Process according to claim 4, in which the said carbonaceous molecular sieve is a stationary bed of particles, vinylidene chloride is a gas and the hourly gas space velocity of the said vinylidene chloride is from approximately 130 to 1500 $h^{-1}$.

6. Process according to claim 4, in which the said carbonaceous molecular sieve is a stationary bed of particles, vinylidene chloride is a liquid and the hourly liquid space velocity of the said vinylidene chloride is from approximately 1 to 15 $h^{-1}$.

**Patentansprüche**

1. Verfahren zur Reinigung von 1,1-Dichlor-1-fluorethan (CFC-141b), das etwa 4 bis 20 ppm Dichloracetylen an Gewicht enthält, wobei man das genannte 1,1-Dichlor-1-fluorethan über ein kohlenstoffhaltiges Molekularsieb mit einer mittleren Porengröße zwischen etwa 4,2 und 4,5 Angström bei einer Temperatur von -20°C bis 60°C und einem Druck von etwa 100 bis 500 kPa leitet und das 1,1-Dichlor-1-fluorethan mit einem Gehalt von weniger als 2 ppm Dichloracetylen an Gewicht gewinnt.

2. Verfahren nach Anspruch 1, in dem das genannte kohlenstoff-haltige Molekularsieb ein festes Teilchenbett ist, das 1,1-Dichlor-1-fluorethan ein Gas ist, und die stündliche Gasdurchgangsgeschwindigkeit des genannten 1,1-Dichlor-1-fluo-rethans etwa 130 bis 1500 $h^{-1}$ beträgt.

3. Verfahren nach Anspruch 1, in dem das genannte kohlenstoff-haltige Molekularsieb ein festes Teilchenbett ist, das 1,1-Dichlor-1-fluorethan eine Flüssigkeit ist, und die stündliche Flüssigkeitsdurchgangsgeschwindigkeit des genannten 1,1-Dichlor-1-fluorethans etwa 1 bis 15 $h^{-1}$ beträgt.

4. Verfahren zur Reinigung von Vinylidenchlorid, das etwa 4 bis 20 ppm Dichloracetylen an Gewicht enthält, wobei das genannte Vinylidenchlorid über ein kohlenstoffhaltiges Molekularsieb mit einer mittleren Porengröße zwischen etwa 4,2 und 4,5 Angström bei iner Temperature von -20°C bis 60°C und einem Druck von etwa 100 bis 500 kPa leitet und das Vinylidenchloride mit einen Gehalt von weniger als 2 ppm Dichloracetylen an Gewicht gewinnt.

5. Verfahren nach Anspruch 4, in dem das genannte kohlenstoffhaltige Molekularsieb ein festes Teilchenbett ist, das Vinylidenchlorid ein Gas ist, und die stündliche Gasdurchgangsgeschwindigkeit des genannten Vinylidenchlorid etwa 130 bis 1500 $h^{-1}$ beträgt.

6. Verfahren nach Anspruch 4, in dem das genannte kohlenstoff-haltige Molekularsieb ein festes Teilchenbett ist, das Vinylidenchlorid eine Flüssigkeit ist, und die stündliche Flüssigkeitsdurchgangsgeschwindigkeit des genannten Vinylidenchlorid etwa 1 bis 15 $h^{-1}$ beträgt

EP 0 490 003 B1